# EUROPÄISCHE PATENTSCHRIFT

(11) **EP 0 561 263 B1**
(45) Veröffentlichungstag und Bekanntmachung des Hinweises auf die Patenterteilung: **15.05.1996**
(21) Anmeldenummer: 93103803.8
(22) Anmeldetag: 10.03.1993
(51) Int. Cl.: A61F 2/30, A61L 27/00

(54) **Implantat als Knochenersatz**
Bone replacement implant
Implant de remplacement d'os

(30) Priorität: 14.03.1992 DE 4208247
(43) Veröffentlichungstag der Anmeldung: 22.09.1993
(73) Patentinhaber: ESKA medical GmbH & Co., D-23556 Lübeck (DE)
(72) Erfinder: Gradinger, Rainer, Prof. Dr. med., W-8000 München 2 (DE); Grundei, Hans, W-2400 Lübeck 1 (DE)
(74) Vertreter: Fuchs, Luderschmidt & Partner Patentanwälte

(56) Entgegenhaltungen:
- EP-A- 0 159 036
- EP-A- 0 337 757
- DE-A- 3 224 265
- DE-C- 3 917 033
- DE-C- 4 106 971
- DE-U- 8 607 873
- US-A- 4 542 539
- US-A- 4 865 608

## Beschreibung

Die Erfindung betrifft ein Implantat als Ersatz für Knochen, insbesondere als Gelenkersatz mit einer seine Oberfläche zumindest teilweise bedeckenden offenmaschigen dreidimensionalen Struktur.

Derartige Implantate sind seit etwa 10 Jahren im klinischen Einsatz. Im Gegensatz zu zementierbaren Implantaten erfolgt die Fixation der eingangs genannten Implantate zementlos, dadurch, daß in die dreidimensionale Struktur Knochentrapekel einwachsen und dort verknöchern. Die dreidimensionale Oberflächenstruktur derartiger Implantate wird also regelrecht durchwachsen durch Knochenmaterial, was zu im Prinzip hervorragenden Ergebnissen bei der Langzeitfixation hervorruft. Implantate dieser Art sind beispielsweise bekannt geworden aus der DE-A1-29 10 627, der DE-A1-32 24 265 und in jüngerer Zeit aus der DE-U1-90 11 363.

Wenn die Langzeitfixation - wie bereits erwähnt - im Prinzip die an sie gestellten Erwartungen auch erfüllt, so weisen die bekannten Implantate jedoch den Nachteil auf, daß sie nur in Grenzen eine funktionale Einheit mit dem sie umgebenden Knochenmaterial bilden, in dem Sinne, daß aufgrund der unterschiedlichen Knochendichtestruktur in Bereichen des Implantates unterschiedliche Elastizitätsmoduln in der natürlichen Spongiosa des Knochens einer recht einheitlichen Oberflächenstruktur des Implantates gegenüberstehen. In bestimmten Bereichen der Knochenspongiosa eines Röhrenknochens ist die Spongiosa von einer relativ großen Zellig- und Porigkeit zu den Gelenkenden hin, wohingegen es Bereiche gibt, wo die Zell- und Porengröße der Knochenspongiosa etwa um einen Faktor bis zu 3 kleiner ist als in dem erstgenannten Bereich. Als Beispiel für einen solchen Röhrenknochen wird der menschliche Femur angegeben. Es ist einleuchtend, daß die Knochentrapekel als Bestandteil der natürlichen Spongiosa in homogener Zellig- und Porigkeit nicht sämtlich gleich gut in die einheitliche Oberflächenstruktur des Implantates einwachsen können. Letztlich führt dies zu einer Verlängerung der Einheilphase und Umbauphase des knöchernen Lagers zum Implantat hin und zu einer Verkürzung der Langzeitfixation aufgrund der Tatsache, daß sozusagen Angehörige einer Knochentrapekelgruppe in die Oberflächenstruktur einwachsen, die nicht der Gruppe der an einer bestimmten Stelle der Spongiosa vorherrschenden Zelligkeit und Porigkeit zuzuordnen sind.

Vor diesem Hintergrund ist es die Aufgabe der vorliegenden Erfindung, ein Implantat mit einer seine Oberfläche zumindest teilweise bedeckenden offenmaschigen dreidimensionalen Struktur zu schaffen, deren Einheilverhalten sowie deren Langzeitfixationseigenschaften deutlich gegenüber bekannten Implantaten verbessert sind.

Gelöst wird die Aufgabe durch das Implantat mit den Merkmalen des Anspruchs 1. Weitere vorteilhafte Ausgestaltungen ergeben aus den abhängigen Ansprüchen.

Demgemäß wird vorgeschlagen, daß die Oberflächenstruktur des Implantates in ihrer flächigen Ausdehnung in zumindest zwei diskrete Zonen unterschiedlicher Maschenweiten unterteilt ist. Bei dieser Festlegung wird unter flächiger Ausdehnung ein Oberflächenabschnitt des Implantates verstanden im Gegensatz zu der räumlichen Ausdehnung, welche auf die Dicke oder Stärke der Struktur abzielt.

Die Maschenweiten der Zonen sind vorzugsweise der Zelligkeit und der Porigkeit des Knochenmaterials, welches nach der Implantation mit der Oberflächenstruktur des Implantates in Verbindung steht, angepaßt, in dem Sinne, daß die jeweiligen Maschenweiten der Zonen in etwa der Größe der Zellen und Poren der natürlichen Spongiosa entsprechen, welche in und an die Oberflächenstruktur des Implantates einwachsen soll. Für die Spongiosa mit einer kleinen Zell- und Porenweite wird demnach auf dem Implantat eine Struktur mit einer entsprechend kleinen Maschenweite vorgesehen. Umgekehrt wird für eine Spongiosa mit einer großen Zell- und Porenweite eine Zone auf dem Implantat mit einer entsprechend großen Maschenweite vorgesehen.

Die Zell- und Porenweiten der natürlichen Spongiosa in Röhrenknochen des Menschen ist katalogisierbar, so daß die Implantate in Anwendung dieses Katalogs mit entsprechenden Zonen unterschiedlicher Maschenweite hergestellt werden können, je nachdem, für welche Knochenpartie das Implantat bestimmt ist.

Die erfindungsgemäße Ausbildung des Implantates hat zur Folge, daß Knochentrapekel des spongiösen Knochens mit einer gegebenen Zell- und Porengröße in die Oberflächenstruktur mit einer entsprechenden Maschenweite einwachsen können. Die Knochentrapekel treffen auf quasi natürliche Gegebenheiten, so daß ein deutlich besseres Einwachsverhalten der Implantate beobachtet werden kann. Darüber hinaus läßt das erfindungsgemäße Implantat verbesserte Eigenschaften im Hinblick auf eine Langzeitfixation erkennen, da die "richtigen" Knochentrapekel in die "richtige" Maschenweite der Struktur einwachsen können und so die Elastizitätsmoduln in der natürlichen Spongiosa weitgehend erhalten bleiben können trotz der Anwesenheit des Implantates.

Die größte Maschenweite einer Zone der Oberflächenstruktur beträgt 6 mm, die kleinste Maschenweite einer Zone 1,5 mm. Sämtliche Bereiche zwischen diesen beiden Grenzwerten können vorgesehen sein in Abhängigkeit von der natürlichen Ausbildung der Spongiosa des Knochens, an oder in den das Implantat eingesetzt werden soll.

Gemäß einer bevorzugten Ausführungsform sind mindestens drei Zonen der Oberflächenstruktur vorgesehen, deren Maschenweiten sich, ausgehend von der größten, stufenförmig von Zone zu Zone vermindern. Ein konkretes Implantat, welches so ausgebildet sein kann, ist ein Hüftgelenksstiel. Entsprechend der natürlichen Spongiosa im Femurknochen ist im proximalen Bereich des Implantates eine großen Maschenweite von bis zu 6 mm vorhanden, der sich eine mittlere Zone mit einer Maschenweite von 2-4 mm anschließt, welcher sich distalseitig einer Zone anschließt, deren Maschenweite bis zu 1,5 mm herunter reichen kann.

Gemäß einer vorteilhaften Weiterbildung des Implantates sind in den Übergangsbereichen von einer Zone zu der benachbarten Zone der Oberflächenstruktur Maschenweiten beider angrenzenden Zonen vorhanden, d. h. daß also in der räumlichen Tiefe der Oberflächenstruktur sowohl die eine als auch die andere Maschenweite präsent ist. Dies wird freilich nur bei einem relativ kleinen Übergangsbereich der Fall sein. Durch diese Ausbildung ergeben sich zwar immer noch diskrete Zonen der Struktur mit unterschiedlichen Maschenweiten, die Übergänge werden aber quasi vergleichmäßigt im Sinne eines und im Hinblick auf einen quasi stetigen Übergang von einer Zone in die andere. Dies entspricht weitgehend dem allmählichen Wechsel des natürlichen spongiösen Materials von einer Zell- und Porenweite zu einer anderen Zell- und Porenweite.

Die Erfindung wird anhand eines Ausführungsbeispiels gemäß der Zeichnungsfiguren näher erläutert. Hierbei zeigt:
- Fig. 1: ein Implantat als Hüftgelenksstiel mit einer ihn teilweise bedeckenden dreidimensionalen offenmaschigen Struktur,
- Fig. 2: eine schematisierte Ansicht mehrerer Zonen der Oberflächenstruktur eines Implantates nach der Erfindung, und
- Fig. 3: eine ähnliche Ansicht wie Fig. 2, aber mit einer anders aufgebauten Oberflächenstruktur.

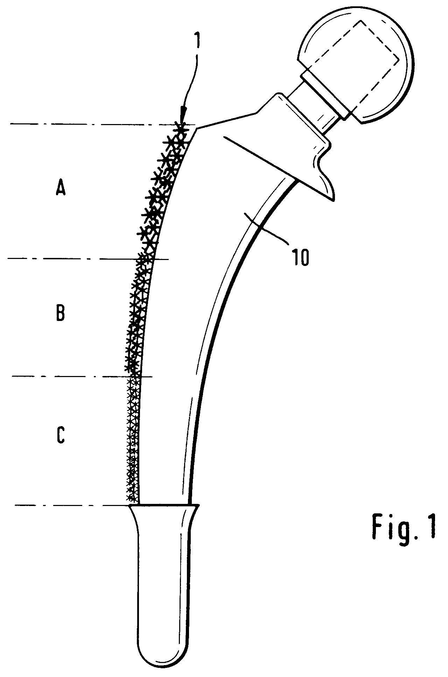

Das dargestellte Ausführungsbeispiel des erfindungsgemäßen Implantats gemäß Fig. 1 stellt einen üblichen Hüftgelenksstiel 10 dar. Der massive Kern des Stiels 10 ist, wie schematisch dargestellt, mit einer seine Oberfläche zumindest teiweise bedeckenden offenmaschigen dreidimensionalen Struktur 1 versehen. In diese Oberflächenstruktur wächst nach der Implantation Knochenmaterial ein und sorgt so für eine Dauerfixation des Implantats im Knochen.

Erfindungsgemäß ist die Oberflächenstruktur 1 des Hüftgelenksstiels 10 unterteilt in drei diskrete Zonen A, B und C. In der Zone A zeigt die Oberflächenstruktur die größte Maschenweite, in der Zone B eine mittlere Maschenweite und schließlich in der Zone C die kleinste Maschenweite. Im dargestellten Ausführungsbeispiel sind die Maschenweiten gebildet aus Partikeln, wie sie in der DE-C1-41 06 971 beschrieben sind. Es kann auch ein anderes Material zur Erzeugung der Oberflächenstrukturen verwendet werden, beispielsweise präparierte Filterschäume, wie in der DE-C1-39 17 033 und der DE-C2-39 28 394 beschrieben ist.

Die Auswahl der Maschenweiten der Oberflächenstruktur 1 in den Zonen A, B und C richtet sich nach den natürlichen Zellen- und Porenweiten der Spongiosa im Femurknochen. Die Maschenweiten der Oberflächenstruktur 1 sind den Zell- und Porenweiten der Spongiosa angepaßt, d. h. im proximalen Bereich zum Hüftgelenksende hin relativ groß, im distalen Bereich relativ klein.

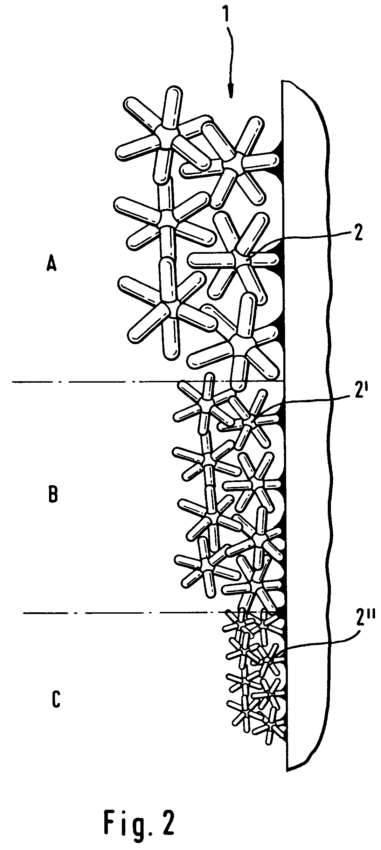

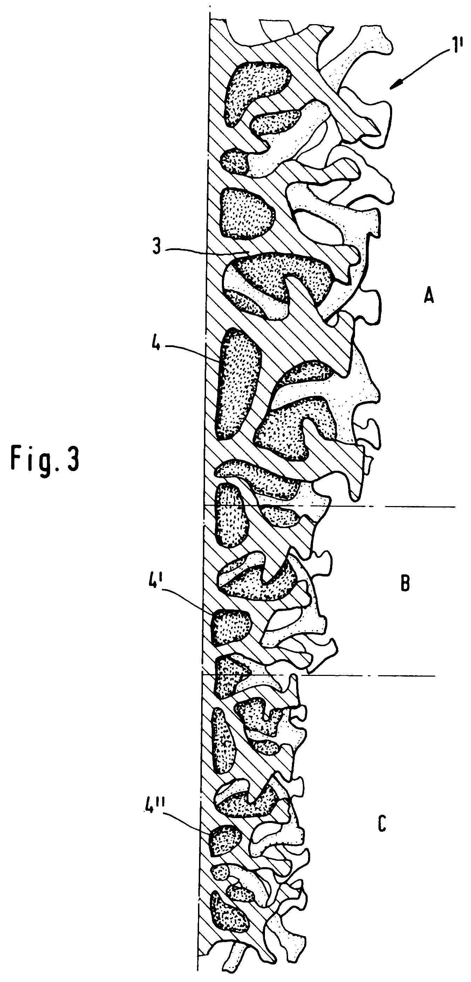

Fig. 2 zeigt schematisiert eine vergrößerte Ansicht der Oberflächenstruktur 1 des erfindungsgemäßen Implantats. Deutlich erkennbar sind die diskreten Zonen A, B und C mit unterschiedlichen Maschenweiten der Oberflächenstruktur 1. Diese werden erzielt durch die Partikel 2, 2' und 2'' unterschiedlicher Größe. Wie bereits weiter oben erwähnt, können diese unterschiedlichen Maschenweiten anstelle durch unterschiedlich große Partikel auch durch retikulierte Filterschäume mit unterschiedlichen Maschenweiten als Ausgangsmaterial im Herstellungsverfahren verwendet werden. Eine Oberflächenstruktur 1' eines Implantats, hergestellt mit retikulierten Filterschäumen als Ausgangsmaterial eines Gießverfahrens (DE-C2-39 28 394), zeigt Fig. 3. Auch hier sind deutlich die diskreten Zonen A, B und C erkennbar, die unterschiedlich große, von den Stegen 3 eingefaßte Maschen 4, 4' und 4'' aufweisen.

Daß die Oberflächenstrukturen 1 und 1' in ihren Stärken mit der Abnahme der Größe der Partikel in den diskreten Zonen A, B und C ebenfalls abnehmen, ist nicht zwingend erforderlich. Wesentlich ist, daß die Knochentrapekel der betreffenden Spongiosabereiche des Knochens, an denen die Zonen A, B und C jeweils zu liegen kommen, Maschenweiten in der Oberflächenstruktur 1 auffinden, in die sie hineinwachsen sollen und die in ihrer Maschenweite in etwa der Zellen- und Porengröße entsprechen, welche die jeweiligen Knochentrapekel in der natürlichen Spongiosa ausbilden würden.

## Patentansprüche

1. Implantat als Ersatz für Knochen mit einer seine Oberfläche zumindest teilweise bedeckenden offenmaschigen dreidimensionalen Struktur (1, 1'), bei dem die Struktur in ihrer flächigen Ausdehnung in zumindest zwei diskrete Zonen (A, B, C) unterschiedlicher Maschenweiten unterteilt ist.

2. Implantat nach Anspruch 1, bei dem die größte Maschenweite einer Zone der Struktur (1, 1') 6 mm und die kleinste Maschenweite einer Zone 1,5 mm beträgt.

3. Implantat nach Anspruch 1 oder 2, bei dem mindestens drei Zonen der Oberflächenstruktur (1, 1') vorgesehen sind, deren Maschenweiten sich, ausgehend von der größten, stufenförmig von Zone zu Zone vermindern.

4. Implantat nach einem der Ansprüche 1 bis 3, bei dem in den Übergangsbereichen von einer Zone (A bzw. B) zu der benachbarten Zone (B bzw. C) der Oberflächenstruktur (1) Maschenweiten beider angrenzender Zonen (A und B bzw. B und C) vorhanden sind.

## Claims

1. Implant as a bone replacement having an open-mesh three-dimensional structure (1, 1') at least partly covering its surface, in which the surface expansion of the structure is divided into at least two discrete zones (A, B, C) of different mesh widths.

2. Implant according to claim 1, in which the largest mesh width of a zone of the structure (1, 1') is 6 mm and the smallest mesh width of a zone is 1.5 mm.

3. Implant according to claim 1 or 2, in which at least three zones are provided in the surface structure (1, 1'), the mesh widths of which decrease gradually from zone to zone starting from the largest.

4. Implant according to one of claims 1 to 3, in which mesh widths of both bordering zones (A and B or B and C) are present in the transition regions from one zone (A or B) to the neighbouring zone (B or C) in the surface structure (1).

## Revendications

1. Implant servant à remplacer des os, présentant une structure (1, 1') tridimensionnelle à mailles ouvertes recouvrant, au moins partiellement, sa surface, dans lequel la structure, dans sa dimension plane, est divisée en au moins deux zones discrètes (A, B, C) de différentes largeurs de mailles.

2. Implant selon la revendication 1, dans lequel la plus grande largeur de mailles d'une zone de la structure (1, 1') est de 6 mm, et la plus faible largeur de mailles d'une zone est de 1,5 mm.

3. Implant selon la revendication 1 ou 2, dans lequel il est prévu au moins trois zones de la structure superficielle (1, 1'), dont les largeurs de mailles vont en se réduisant par échelons, de zone en zone, en commençant par la plus importante.

4. Implant selon l'une des revendications 1 à 3, dans lequel dans les zones de transition entre une zone (A respectivement B) et la zone voisine (B respectivement C) de la structure superficielle (1), on trouve des largeurs de mailles des deux zones adjacentes (A et B respectivement B et C).
